# EUROPÄISCHE PATENTSCHRIFT

(11) **EP 4 282 349 B1**
(45) Veröffentlichungstag und Bekanntmachung des Hinweises auf die Patenterteilung: **27.08.2025**
(21) Anmeldenummer: 23174548.0
(22) Anmeldetag: 22.05.2023
(51) Int. Cl.: A61B 17/15, A61B 17/56, A61F 2/38, A61F 2/46

(54) **CHIRURGISCHES INSTRUMENTENSYSTEM**
SURGICAL INSTRUMENT SYSTEM
SYSTÈME D'INSTRUMENT CHIRURGICAL

(30) Priorität: 24.05.2022 DE 102022205192
(43) Veröffentlichungstag der Anmeldung: 29.11.2023
(73) Patentinhaber: Aesculap AG, 78532 Tuttlingen (DE)
(72) Erfinder: Firmbach, Franz-Peter, 78576 Emmingen-Liptingen (DE); Anhorn, Svenja, 72535 Heroldstatt (DE)
(74) Vertreter: Patentanwälte Ruff, Wilhelm, Beier, Dauster & Partner mbB

(56) Entgegenhaltungen:
- WO-A1-2018/216026
- WO-A1-95/14444
- WO-A1-99/20192
- DE-C1- 4 339 895

## Beschreibung

Die Erfindung betrifft ein chirurgisches Instrumentensystem mit den Merkmalen des Oberbegriffs des Anspruchs 1.

Aus der WO 2018/216026 A1 ist ein chirurgisches Instrumentensystem mit den oberbegrifflichen Merkmalen des Anspruch 1 bekannt.

Ein weiteres chirurgisches Instrumentensystem ist aus der US 10,130,375 B2 bekannt und zur Verwendung bei einer totalen Kniegelenkersatzoperation (englisch: total knee arthroplasty, abgekürzt TKA) vorgesehen. Das bekannte chirurgische Instrumentensystem weist einen Referenzblock und wenigstens ein als Shim bezeichnetes Kompensationselement auf. Der Referenzblock ist zur Anbringung an einem distalen Femur eingerichtet und weist eine Blockvorderseite sowie eine gegenüberliegende Blockrückseite auf. In der Verwendung ist die Blockvorderseite den distalen Kondylen des Femurs zugewandt. Das Kompensationselement weist eine Elementrückseite und eine gegenüberliegende Kontaktfläche auf. Die Kontaktfläche ist zur Kontaktierung einer der distalen femoralen Kondylen eingerichtet. Bei dem bekannten chirurgischen Instrumentensystem ist die Kontaktfläche kongruent mit der Blockvorderseite, so dass letztere in angebrachtem Zustand des Kompensationselements vollständig überdeckt ist. Zur lösbaren Anbringung des Kompensationselements weist der Referenzblock eine Art Steckaufnahme auf. Das Kompensationselement ist mit einem rückseitigen Vorsprung versehen, der lösbar in die Steckaufnahme einsteckbar ist. Die Steckaufnahme und der Vorsprung bilden eine Art Verbindungssystem.

Aufgabe der Erfindung ist es, ein chirurgisches Instrumentensystem der eingangs genannten Art bereitzustellen, welches eine an den jeweiligen Patienten angepasste und besonders flexible Verwendung bei gleichzeitig einfachem Aufbau erlaubt und letztlich verbesserte Operationsergebnisse ermöglicht.

Diese Aufgabe wird durch das Bereitstellen eines chirurgischen Instrumentensystems mit den Merkmalen des Anspruchs 1 gelöst. Die erfindungsgemäße Lösung ermöglicht eine angepasste Positionierung des Kompensationselements im Hinblick auf eine Position und/oder örtliche Ausprägung einer mittels des Kompensationselements zu kompensierenden kondylären Knochen- und/oder Knorpelabnutzung. Die unterschiedliche Positionierung des Kompensationselements an dem Referenzblock erfolgt insoweit in Abhängigkeit der Größe des zu kompensierenden Defekts und/oder in Abhängigkeit davon, ob der zu kompensierende Defekt in einem in Bezug auf die Anterior-Posterior-Achse oberen, mittigen oder unteren Bereich an der medialen und/oder lateralen Kondyle des distalen Femurs verortet ist. Zu diesem Zweck ist das Verbindungssystem erfindungsgemäß derart eingerichtet, dass das Kompensationselement in unterschiedlichen Positionen entlang der Anterior-Posterior-Achse auf der Blockvorderseite anbringbar ist. Da das Kompensationselement die Blockvorderseite in angebrachtem Zustand wenigstens in Bezug auf die Anterior-Posterior-Achse nicht vollständig überdeckt, wird gleichzeitig - unabhängig von der jeweils eingenommenen Position des Kompensationselements - eine möglichst uneingeschränkte Sicht des Operateurs auf die Kontaktstelle zwischen der Kontaktfläche und der jeweiligen Kondyle ermöglicht. Durch die erfindungsgemäße Lösung ist das chirurgische Instrumentensystem besonders flexibel verwendbar und an die Geometrie und Lage der zu kompensierenden kondylären Abnutzung anpassbar. In Kombination mit der gleichzeitig verbesserten oder jedenfalls nicht beeinträchtigen Einsehbarkeit der Kontaktstelle werden letztlich verbesserte Operationsergebnisse ermöglicht.

Der Referenzblock ist vorzugsweise zur intramedullären Anbringung an dem distalen Femur eingerichtet. Zu diesem Zweck weist der Referenzblock vorzugsweise einen zwischen der Blockvorderseite und der Blockrückseite erstreckten Durchlass auf, welcher zur Aufnahme eines intramedullären Stabs eingerichtet ist. Eine solche intramedulläre Anbringung ist dem Fachmann grundsätzlich bekannt und bedarf daher keiner weitergehenden Erläuterung. Alternativ oder zusätzlich kann der Referenzblock zur extramedullären Anbringung eingerichtet sein. Dies ebenfalls auf eine dem Fachmann grundsätzlich bekannte Weise. In der Verwendung des chirurgischen Instrumentensystems ist die Blockvorderseite dem distalen Femur zugewandt und insoweit proximal orientiert. Die gegenüberliegende Blockrückseite ist dementsprechend distal orientiert. Vorzugsweise ist die Blockrückseite zur lösbaren Anbringung eines Femursägeblocks (englisch: femoral cut guide) eingerichtet. In angebrachtem Zustand und nach erfolgter Kompensation der kondylären Abnutzung mittels des wenigstens einen Kompensationselements kann der Referenzblock eine Referenz für unter Verwendung des Femursägeblocks auszuführende Sägeschnitte an dem distalen Femur dienen. Der Referenzblock weist vorzugsweise die Proximal-Distal-Achse, die Anterior-Posterior-Achse und eine Medial-Lateral-Achse auf, die jeweils orthogonal zueinander ausgerichtet sind und somit ein kartesisches Bezugsachsensystem bilden. Zur Vollständigkeit sei angemerkt, dass die besagten Achsen des Referenzblocks in der Verwendung des chirurgischen Instrumentensystems vorzugsweise mit den entsprechenden Körperachsen des Patienten korrespondieren. Mit anderen Worten ausgedrückt, kann die Anterior-Posterior-Achse auch als Hochachse, die Medial-Lateral-Achse als Querachse und die Proximal-Distal-Achse als Längsachse des Referenzblocks bezeichnet werden. Bei Ausgestaltungen der Erfindung ist der Referenzblock einstückig ausgebildet. Bei anderen Ausgestaltungen ist der Referenzblock mehrteilig ausgeführt.

Das wenigstens eine Kompensationselement ist in der Verwendung des chirurgischen Instrumentensystems mit seiner Elementrückseite der Blockvorderseite zugewandt. Die gegenüberliegende Kontaktfläche ist dem distalen Femur zugewandt. In angebrachtem Zustand des Kompensationselements ist eine Proximal-Distal-Achse des Kompensationselements vorzugsweise parallel zu der Proximal-Distal-Achse des Referenzblocks ausgerichtet. In dem angebrachten Zustand überdeckt das Kompensationselement die Blockvorderseite nicht vollständig und/oder unvollständig. Die besagte Überdeckung ist wenigstens in Bezug auf die Anterior-Posterior-Achse, also in Hochrichtung des Referenzblocks, nicht vollständig. Bevorzugt ist die Überdeckung zusätzlich in Bezug auf die Medial-Lateral-Achse des Referenzblocks, d.h. in Querrichtung des Referenzblocks, unvollständig. Mit anderen Worten ausgedrückt, ist das Kompensationselement weniger hoch und gegebenenfalls zusätzlich weniger breit als die Blockvorderseite. Hierdurch kann das Kompensationselement in den unterschiedlichen Positionen auf der Blockvorderseite positioniert werden, ohne dass eine Umfangskontur des Kompensationselements über eine Umfangskontur der Blockvorderseite hinausragt. Dies unterstützt die besagte verbesserte Einsehbarkeit der Kontaktstelle. Das wenigstens eine Kompensationselement weist eine definierte Dicke entlang der Proximal-Distal-Achse auf. Vorzugsweise weist das chirurgische Instrumentensystem mehrere unterschiedliche Kompensationselemente mit unterschiedlichen definierten Dicken auf, die zur Kompensation unterschiedlich stark ausgeprägter kondylärer Knochen- und/oder Knorpelabnutzungen eingerichtet sind. Bei Ausgestaltung der Erfindung ist das wenigstens eine Kompensationselement einstückig ausgebildet. Bei anderen Ausgestaltungen ist das Kompensationselement mehrteilig ausgeführt.

Das Verbindungssystem ist abschnittsweise an dem Referenzblock und abschnittsweise an dem wenigstens einen Kompensationselement angeordnet und/oder ausgebildet. Dementsprechend weist das Verbindungssystem den wenigstens einen an dem Referenzblock angeordneten und/oder ausgebildeten Verbindungsabschnitt und den wenigstens einen an dem Kompensationselement angeordneten und/oder ausgebildeten komplementären Verbindungsabschnitt auf. Sofern das chirurgische Instrumentensystem mehrere Kompensationselemente aufweist, weist jedes der Kompensationselemente wenigstens einen komplementären Verbindungsabschnitt auf, wobei sämtliche komplementären Verbindungsabschnitte dem Verbindungssystem zuzurechnen sind. Zur lösbaren Anbringung des Kompensationselements an dem Referenzblock wirken der wenigstens eine Verbindungsabschnitt und der wenigstens eine komplementäre Verbindungsabschnitt form- und/oder kraftschlüssig zusammen. Die lösbare Verbindung zwischen dem Verbindungsabschnitt und dem komplementären Verbindungsabschnitt ist eine Steckverbindung. Um die Positionierung des Kompensationselements in den unterschiedlichen Positionen zu ermöglichen, sind bei Ausgestaltung der Erfindung beispielsweise mehrere Verbindungsabschnitte voneinander beabstandet an dem Referenzblock angeordnet und/oder ausgebildet. Bei anderen Ausgestaltungen ist beispielsweise lediglich ein Verbindungsabschnitt an dem Referenzblock vorhanden, der in einem mit dem komplementären Verbindungsabschnitt verbundenen Zustand wenigstens einen Rotationsfreiheitsgrad des Kompensationselements in Relation zu dem Referenzblock gestattet. Hierdurch kann das Kompensationselement in einem bereits an der Blockvorderseite angebrachten Zustand in unterschiedliche Positionen gedreht werden.

In Ausgestaltung der Erfindung ist der wenigstens eine Verbindungsabschnitt an der Blockvorderseite angeordnet und der wenigstens eine komplementäre Verbindungsabschnitt ist an der Elementrückseite angeordnet. Eine solche Anordnung kann insbesondere fertigungstechnische und/oder ergonomische Vorteile bei der Anbringung des Kompensationselements mit sich bringen. Eine solche Anordnung ist jedoch nicht zwingend. Bei anderen Ausgestaltungen ist der komplementäre Verbindungsabschnitt an einer Umfangskontur, d.h. seitlich, ober- und/oder unterseitig an dem Kompensationselement angeordnet. Entsprechendes kann sinngemäß für die Anordnung des wenigstens einen Verbindungsabschnitts an dem Referenzblock gelten, wobei zudem eine Anordnung an dessen Blockrückseite denkbar ist.

In weiterer Ausgestaltung der Erfindung ist der wenigstens eine Verbindungsabschnitt ein in den Referenzblock eingesenkter weiblicher Verbindungsabschnitt und der wenigstens eine komplementäre Verbindungsabschnitt ist ein von dem Kompensationselement aufragender männlicher Verbindungsabschnitt. Eine solche Gestaltung kann insbesondere fertigungstechnische Vorteile bei der Herstellung und/oder ergonomische Vorteile bei der Verwendung, im Speziellen bei der Anbringung des Kompensationselements, mit sich bringen. Vorzugsweise ist der wenigstens eine Verbindungsabschnitt in die Blockvorderseite eingesenkt. Vorzugsweise ragt der wenigstens eine komplementäre Verbindungsabschnitt von der Elementrückseite auf. Eine eingesenkte und insoweit weibliche Gestaltung kann insbesondere als Bohrung, Einsenkung, Rücksprung, Nut, Schlitz oder dergleichen ausgeführt sein. Eine aufragende und insoweit männliche Gestaltung kann insbesondere als Zapfen, Stift, Bolzen, Leiste, Haken oder dergleichen ausgeführt sein. Bei weiteren Ausgestaltungen ist eine hierzu umgekehrte Zuordnung von männlichen und weiblichen Abschnitten vorgesehen.

In weiterer Ausgestaltung der Erfindung weist die Blockvorderseite einen medialen Abschnitt und einen entlang einer Medial-Lateral-Achse des Referenzblocks beabstandeten lateralen Abschnitt auf, welche jeweils wenigstens einen Verbindungsabschnitt des Verbindungssystems aufweisen, wobei das wenigstens eine Kompensationselement zur Kontaktierung der lateralen Kondyle auf dem lateralen Abschnitt und zur Kontaktierung der medialen Kondyle auf dem medialen Abschnitt anbringbar ist. Hierdurch wird eine nochmals flexiblere und besonders anpassbare Verwendung ermöglicht. Denn bei dieser Ausgestaltung der Erfindung ist das wenigstens eine Kompensationselement wahlweise zur Kompensation einer Abnutzung der lateralen Kondyle oder der medialen Kondyle eingerichtet. Hierzu kann das Kompensationselement wahlweise entweder auf dem lateralen Abschnitt oder dem medialen Abschnitt angebracht werden. Zu diesem Zweck weist das Verbindungssystem auf beiden Abschnitten der Blockvorderseite wenigstens einen Verbindungsabschnitt auf. Diese Verbindungsabschnitte können dementsprechend auch als lateraler Verbindungsabschnitt bzw. medialer Verbindungsabschnitt bezeichnet werden. Sowohl auf dem medialen Abschnitt als auch auf dem lateralen Abschnitt ist das Kompensationselement wiederum wenigstens in Bezug auf die Anterior-Posterior-Achse unterschiedlich positionierbar.

Weiter gemäß der Erfindung ist der wenigstens eine Verbindungsabschnitt eine in die Blockvorderseite eingebrachte zylindrische Bohrung, und der wenigstens eine komplementäre Verbindungsabschnitt ist ein exzentrisch auf der Elementrückseite angeordneter zylindrischer Zapfen, welcher in angebrachtem Zustand des Kompensationselements axial in die Bohrung eingesteckt ist, wobei das Kompensationselement aufgrund der exzentrischen Anordnung des Zapfens mittels einer Rotation um dessen Längsachse wenigstens in Bezug auf die Anterior-Posterior-Achse unterschiedlich positionierbar ist. Zur unterschiedlichen Positionierung kann das Kompensationselement in unterschiedlichen Orientierungen in Bezug auf die Proximal-Distal-Achse drehbeweglich positioniert werden. Zu diesem Zweck ist zum einen die Bohrung zylindrisch ausgeführt. Zudem ist der Zapfen zylindrisch ausgeführt und exzentrisch auf der Elementrückseite angeordnet. Durch die exzentrische Anordnung des Zapfens bewirkt eine Rotation um die Längsachse des Zapfens nicht lediglich eine unterschiedliche Orientierung, sondern zusätzlich eine Verschiebung des Kompensationselements relativ zu dem Referenzblock. Diese Verschiebung erfolgt entlang der Anterior-Posterior-Achse und/oder der Medial-Lateral-Achse. In einem einfachsten Fall weist die Blockvorderseite lediglich eine einzige zylindrische Bohrung zur Aufnahme des zylindrischen und exzentrisch angeordneten Zapfens auf. Sofern die Blockvorderseite einen medialen Abschnitt und einen lateralen Abschnitt aufweist, ist vorzugsweise jeder der beiden Abschnitte mit einer zylindrischen Bohrung versehen. Diese Ausgestaltung der Erfindung bietet insbesondere fertigungstechnische Vorteile, da lediglich ein Verbindungsabschnitt in Form der zylindrischen Bohrung in die Blockvorderseite eingebracht werden muss.

In weiterer Ausgestaltung der Erfindung weist das Kompensationselement und/oder die Kontaktfläche eine kreisförmige Umfangskontur auf. Die Erfinder haben erkannt, dass die kreisförmige Umfangskontur in Kombination mit der exzentrischen Anordnung des zylindrischen Zapfens auf der Elementrückseite besondere Vorteile bietet.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivdarstellung eine Ausführungsform eines erfindungsgemäßen chirurgischen Instrumentensystems mit einem Referenzblock und wenigstens einem Kompensationselement mit Blickrichtung auf eine Blockvorderseite des Referenzblocks,
- Fig. 2: in schematischer Detaildarstellung das wenigstens eine Kompensationselement des chirurgischen Instrumentensystems nach Fig. 1 mit Blickrichtung auf eine Elementrückseite,
- Fig. 3: eine schematische Perspektivdarstellung des Kompensationselements nach Fig. 2,
- Fig. 4: eine schematische Rückansicht des chirurgischen Instrumentensystems nach Fig. 1,
- Fig. 5: eine schematische Aufsicht des chirurgischen Instrumentensystems nach den Fig. 1 und 4,
- Fig. 6: in schematischer Perspektivdarstellung eine Ausführungsform eines nicht erfindungsgemäßen chirurgischen Instrumentensystems,
- Fig. 7, 8: eine schematische Rückansicht (Fig. 7) und eine perspektivische Rückansicht (Fig. 8) eines Kompensationselements des chirurgischen Instrumentensystems nach Fig. 6,
- Fig. 9, 10: das chirurgische Instrumentensystem nach Fig. 6 in einer schematischen Rückansicht (Fig. 9) und einer schematischen Frontansicht (Fig. 10),
- Fig. 11: in schematischer Perspektivdarstellung eine weitere Ausführungsform eines nicht erfindungsgemäßen chirurgischen Instrumentensystems,
- Fig. 12, 13: ein Kompensationselement des chirurgischen Instrumentensystems nach Fig. 11 in einer schematischen Rückansicht (Fig. 12) und einer perspektivischen Rückansicht (Fig. 13),
- Fig. 14, 15, 16: das chirurgische Instrumentensystem nach Fig. 11 in einer schematischen Rückansicht (Fig. 14), Aufsicht (Fig. 15) und Seitenansicht (Fig. 16),
- Fig. 17: in schematischer Perspektivdarstellung eine weitere Ausführungsform eines nicht erfindungsgemäßen chirurgischen Instrumentensystems,
- Fig. 18, 19: ein Kompensationselement des chirurgischen Instrumentensystems nach Fig. 17 in einer schematischen Rückansicht (Fig. 18) und einer perspektivischen Rückansicht (Fig. 19) und
- Fig. 20, 21: das chirurgische Instrumentensystem nach Fig. 17 in einer schematischen Rückansicht (Fig. 20) und einer schematischen Frontansicht (Fig. 21).

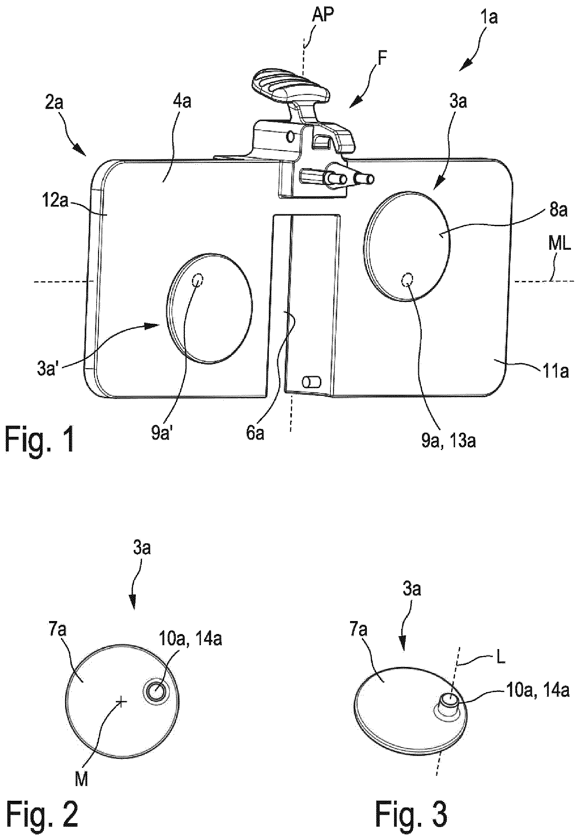

Gemäß Fig. 1 ist ein chirurgisches Instrumentensystem 1a zur Verwendung bei einer Kniegelenkersatzoperation vorgesehen. Im Speziellen dient das chirurgische Instrumentensystem 1a einer Positionierung und/oder Ausrichtung eines sog. Femursägeblocks zur Ausführung von Sägeschnitten an einem distalen Femur. In diesem Zusammenhang erlaubt das chirurgische Instrumentensystem 1a insbesondere eine maßliche Kompensation von kondylären Knochen- und/oder Knorpeldefekten. Das chirurgische Instrumentensystem 1a kann auch als Alignment-System bezeichnet werden.

Das chirurgische Instrumentensystem 1a weist einen Referenzblock 2a und wenigstens ein Kompensationselement 3a auf.

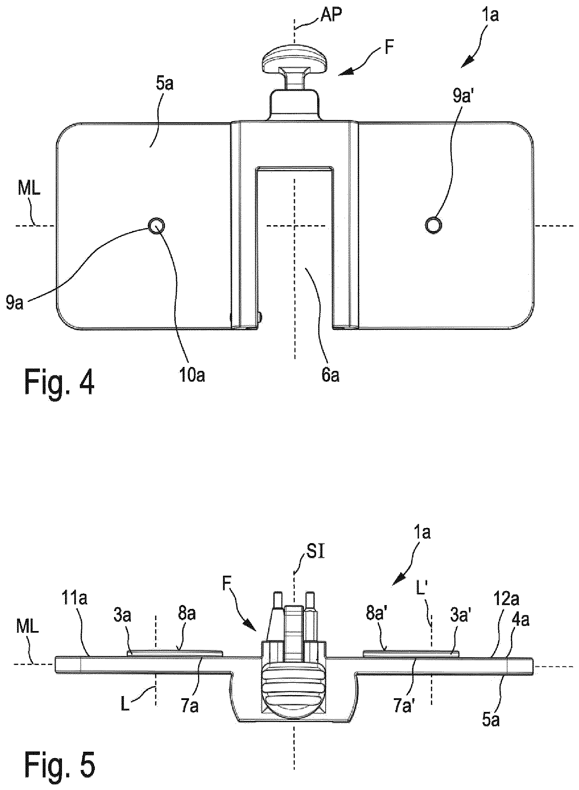

Der Referenzblock 2a ist zur Anbringung an einem vorliegend nicht näher gezeigten distalen Femur eines Patienten eingerichtet und weist eine Blockvorderseite 4a sowie eine entlang einer Proximal-Distal-Achse SI (Fig. 5) gegenüberliegende Blockrückseite 5a (Fig. 4) auf. In der Verwendung des chirurgischen Instrumentensystems 1a ist die Blockvorderseite 4a dem distalen Femur, genauer: dessen Kondylen, zugewandt. Die Blockrückseite 5a ist dem distalen Femur abgewandt.

Die Anbringung des Referenzblocks 2a an dem distalen Femur erfolgt auf eine dem Fachmann grundsätzlich bekannte Weise. Beispielsweise kann eine intramedulläre und/oder extramedulläre Anbringung vorgesehen sein. Zur intramedullären Anbringung weist der Referenzblock 2a bei der gezeigten Ausführungsform einen zwischen der Blockvorderseite 4a und der Blockrückseite 5a durchgängig erstreckten Durchlass 6a auf, welcher zur Aufnahme eines intramedullären Stabs eingerichtet ist. Weitere Details der Anbringung sind für die vorliegende Erfindung nicht von Bedeutung, so dass auf weitere dahingehende Erläuterungen verzichtet werden kann.

Der Referenzblock 2a kann auch als Alignment-Platte (englisch: alignment plate) bezeichnet werden. Bei der gezeigten Ausführungsform weist der Referenzblock 2a eine plattenförmige Gestalt mit in etwa rechteckiger Umfangskontur auf. Der Referenzblock 2a weist neben der besagten Proximal-Distal-Achse SI eine Anterior-Posterior-Achse AP und eine Medial-Lateral-Achse ML auf. Die besagten Achsen SI, AP, ML bilden ein gedachtes kartesisches Achsensystem. Die besagten Achsen SI, AP, ML korrespondieren in der eigentlichen Verwendung des chirurgischen Instrumentensystems 1a vorzugsweise mit der jeweiligen patientenseitigen anatomischen Körperachse.

Die Anterior-Posterior-Achse AP kann - mit Bezug auf die Abmessungsverhältnisse des Referenzblocks 2a - auch als Hochachse und/oder Z-Achse des Referenzblocks 2a bezeichnet werden. Die Medial-Lateral-Achse ML kann auch als Querachse und/oder Y-Achse des Referenzblocks 2a bezeichnet werden. Die Proximal-Distal-Achse SI kann auch als Längsachse und/oder X-Achse des Referenzblocks 2a bezeichnet werden.

Die Blockrückseite 5a ist auf nicht näher gezeigte Weise zur lösbaren Anbringung des bereits erwähnten Femursägeblocks eingerichtet. Die Blockrückseite 5a kann zur Aufnahme zusätzlicher oder alternativer Komponenten eingerichtet sein, so dass eine Verwendung ausschließlich in Kombination mit dem besagten Femursägeblock nicht unbedingt zwingend ist.

Im Übrigen weist der Referenzblock 2a bei der gezeigten Ausführungsform eine Funktionseinheit F auf, die in Bezug auf die Medial-Lateral-Achse ML in etwa mittig und in Bezug auf die Anterior-Posterior-Achse AP oberseitig angeordnet ist. Die Funktionseinheit F ist zur lösbaren Kopplung des Referenzblocks 2a mit weiteren nicht näher bezeichneten Komponenten vorgesehen, was jedoch im Hinblick auf die vorliegende Erfindung nicht von Bedeutung ist. Eine weitere Erläuterung von strukturellen und/oder funktionellen Merkmalen der Funktionseinheit F ist daher vorliegend entbehrlich.

Das wenigstens eine Kompensationselement 3a (siehe insbesondere Fig. 2, 3) weist eine Elementrückseite 7a und eine gegenüberliegende Kontaktfläche 8a auf. Die Kontaktfläche 8a ist zur Kontaktierung des distalen Femurs eingerichtet. Das wenigstens eine Kompensationselement 3a ist auf noch näher beschriebene Weise zur lösbaren Anbringung an der Blockvorderseite 4a eingerichtet. In dem anhand Fig. 1 gezeigten angebrachten Zustand ist die Kontaktfläche 8a parallel zu der Blockvorderseite 4a und derselben abgewandt ausgerichtet. In der Verwendung des chirurgischen Instrumentensystems 1a ist die Kontaktfläche 8a dem distalen Femur zugewandt und liegt entweder an dessen lateraler Kondyle oder medialer Kondyle an. Die Elementrückseite 7a ist in dem angebrachten Zustand des Kompensationselements 3a parallel zu der Blockvorderseite 4a und derselben zugewandt orientiert.

Zur lösbaren Anbringung des Kompensationselements 3a ist ein Verbindungssystem vorgesehen, welches wenigstens einen an dem Referenzblock 2a angeordneten Verbindungsabschnitt 9a und einen an dem Kompensationselement 3a angeordneten komplementären Verbindungsabschnitt 10a aufweist. Das Verbindungssystem ist derart eingerichtet, dass das Kompensationselement 3a mittels des Verbindungssystems wenigstens in Bezug auf die Anterior-Posterior-Achse AP in unterschiedlichen Positionen auf der Blockvorderseite 4a anbringbar ist. Das Kompensationselement 3a überdeckt die Blockvorderseite 4a in dem angebrachten Zustand wenigstens in Bezug auf die Anterior-Posterior-Achse AP nicht vollständig. Mit anderen Worten ausgedrückt, ist das Kompensationselement 3a, im Speziellen dessen Kontaktfläche 8a, weniger hoch als die Blockvorderseite 4a. Vorliegend ist das Kompensationselement 3a und damit auch dessen Kontaktfläche 8a auch in Bezug auf die Medial-Lateral-Achse ML kleiner als die Blockvorderseite, d.h. in Querrichtung weniger breit. Das Kompensationselement 3a kann aufgrund der noch im Detail beschriebenen Gestaltung des Verbindungssystems in einer auf den zu kompensierenden Knorpel- und/oder Knochendefekt angepassten Weise relativ zu der Blockvorderseite 4a positioniert werden. Da das Kompensationselement 3a auf die vorbeschriebene Weise kleiner ist als die Blockvorderseite 4a, ist dennoch eine möglichst freie Einsehbarkeit der Kontaktstelle zwischen der Kontaktfläche 8a und der jeweiligen distalen Kondyle gewährleistet.

Bei der gezeigten Ausführungsform weist die Blockvorderseite 4a einen medialen Abschnitt 11a und einen lateralen Abschnitt 12a auf. Der mediale Abschnitt 11a und der laterale Abschnitt 12a der Blockvorderseite 4a sind vorliegend durch den Durchlass 6a voneinander getrennt und insoweit entlang der medial-lateralen Achse ML voneinander beabstandet. Das Kompensationselement 3a ist in dem anhand Fig. 1 gezeigten Zustand auf dem medialen Abschnitt 11a angebracht. Zu diesem Zweck weist der mediale Abschnitt 11a den Verbindungsabschnitt 9a auf. Zur alternativen Anbringung des Kompensationselements 3a auf dem lateralen Abschnitt 12a weist dieser vorliegend einen weiteren Verbindungsabschnitt 9a' des Verbindungssystems auf. Das Kompensationselement 3a kann demnach wahlweise auf dem medialen Abschnitt 11a oder auf dem lateralen Abschnitt 12a aufgebracht werden. Dies in Abhängigkeit davon, ob ein Knorpel- und/oder Knochendefekt an der medialen Kondyle oder der lateralen Kondyle kompensiert werden muss.

Im Übrigen weist das chirurgische Instrumentensystem 1a bevorzugt eine Mehrzahl von Kompensationselementen auf, welche sich in erster Linie hinsichtlich ihrer Dicke in Bezug auf die Proximal-Distal-Achse unterscheiden können. Denkbar sind beispielsweise unterschiedliche Dicken der unterschiedlichen Kompensationselemente von 1 mm, 2 mm, 3 mm, 4 mm usw. Üblicherweise richtet sich die Dicke des zu verwendenden Kompensationselements nach einem Grad der Ausprägung des Knochen- oder Knorpeldefekts bzw. einem Abnutzungsgrad der Kondyle.

Bei der gezeigten Ausführungsform ist der Referenzblock 2a derart eingerichtet, dass die Blockvorderseite 4a als Kompensationsfläche für eine nicht oder in praktisch unerheblichem Maße abgenutzte Kondyle fungieren kann. D.h. sofern keine merkliche Abnutzung vorliegt, ist die Anbringung eines Kompensationselements nicht erforderlich.

Anhand Fig. 1 ist das chirurgische Instrumentensystem 1a in einem Zustand gezeigt, in welchem zusätzlich zu dem Kompensationselement 3a ein weiteres Kompensationselement 3a' auf der Blockvorderseite 4a angebracht ist. Das weitere Kompensationselement 3a' ist vorliegend auf dem lateralen Abschnitt 12a angebracht. Bei der gezeigten Konfiguration ist das weitere Kompensationselement 3a' baulich identisch mit dem Kompensationselement 3a, so dass auf weitere diesbezügliche Erläuterungen verzichtet werden kann. Es versteht sich, dass anstelle des weiteren Kompensationselements 3a beispielsweise ein solches mit einer unterschiedlichen Dicke verwendet werden kann.

Die weiteren Erläuterungen im Hinblick auf das Verbindungssystem und damit auf den Verbindungsabschnitt 9a sowie den komplementären Verbindungsabschnitt 10a erfolgen anhand des Kompensationselements 3a und des medialen Abschnitts 11a der Blockvorderseite. Es versteht sich, dass die diesbezüglichen Erläuterungen ohne weiteres auf das weitere Kompensationselement 3a' und den lateralen Abschnitt 12a mit dem dort angeordneten weiteren Verbindungsabschnitt 9a' übertragbar sind.

Bei der Ausführungsform nach den Fig. 1 bis 5 ist der Verbindungsabschnitt 9a eine zylindrische Bohrung 13a und der komplementäre Verbindungsabschnitt 10a ist ein zylindrischer Zapfen 14a, welcher exzentrisch auf der Elementrückseite 7a angeordnet ist. Hierdurch ist das Kompensationselement 3a in dem anhand Fig. 1 gezeigten angebrachten Zustand mittels einer Rotation um eine Längsachse L (Fig. 3) des zylindrischen Zapfens 14a in unterschiedlichen Positionen relativ zu der Blockvorderseite 4a an dem Referenzblock 2a positionierbar. Durch die besagte Rotation um die Längsachse L ändert sich die Orientierung des Kompensationselements 3a und somit - aufgrund der exzentrischen Anordnung des zylindrischen Zapfens 14a - auch die Positionierung in Bezug auf die Anterior-Posterior-Achse. Vorliegend wird zudem die Position in Bezug auf die Medial-Lateral-Achse ML geändert. Zur Anbringung des Kompensationselements 3a wird der zylindrische Zapfen 14a entlang seiner Längsachse L, welche parallel zu der Proximal-Distal-Achse SI orientiert ist, in die zylindrische Bohrung 13a eingesteckt. In dem eingesteckten Zustand ist der zylindrische Zapfen 14a in radialer Richtung formschlüssig in der zylindrischen Bohrung 13a gehalten. Der zylindrische Zapfen 14a ist insoweit unbeweglich in Bezug auf die Anterior-Posterior-Achse AP und die Medial-Lateral-Achse ML. Zur unterschiedlichen Positionierung wird das Kompensationselement 3a um die Längsachse L gedreht, wobei der zylindrische Zapfen 14a in Umfangsrichtung relativ zu der zylindrischen Bohrung 13a gleitet. Hierdurch kann die Kontaktfläche 8a an die jeweilige Position der zu kompensierenden kondylären Abnutzung angepasst weiter nach oben, unten, innen und/oder außen positioniert werden.

Bei der gezeigten Ausführungsform weist das Kompensationselement 3a eine kreisförmige Umfangskontur U auf (Fig. 2, 3). Entsprechendes gilt sinngemäß im Hinblick auf die Kontaktfläche 8a. Die kreisförmige Umfangskontur U bietet insbesondere im Zusammenhang mit der vorliegenden Gestaltung des Verbindungssystems zur drehbeweglichen Positionierung besondere Vorteile. Die kreiszylindrische Umfangskontur U weist einen gedachten Mittelpunkt M auf. Der zylindrische Zapfen 14a ist abseits des Mittelpunkts M und insoweit exzentrisch angeordnet.

Bei der anhand der Fig. 1 bis 5 gezeigten Ausführungsform ist der Verbindungsabschnitt 9a als weiblicher Verbindungsabschnitt in Form der zylindrischen Bohrung 13a in die Blockvorderseite 4a eingesenkt. Der komplementäre Verbindungsabschnitt 10a ist hierzu korrespondierend als männlicher Verbindungsabschnitt in Form des zylindrischen Zapfens 14a ausgebildet und ragt axial von der Elementrückseite 7a auf. Bei einer abgewandelten Ausführungsform kann die Anordnung von weiblichem und männlichem Verbindungsabschnitt hierzu umgekehrt sein. Dementsprechend kann der Referenzblock einen, vorzugsweise entfernbaren, zylindrischen Zapfen und das Kompensationselement eine zylindrische Bohrung aufweisen. Dementsprechend ist die anhand der Fig. 1 bis 5 gezeigte Zuordnung von männlichem und weiblichem Verbindungsabschnitt nicht als zwingend zu erachten.

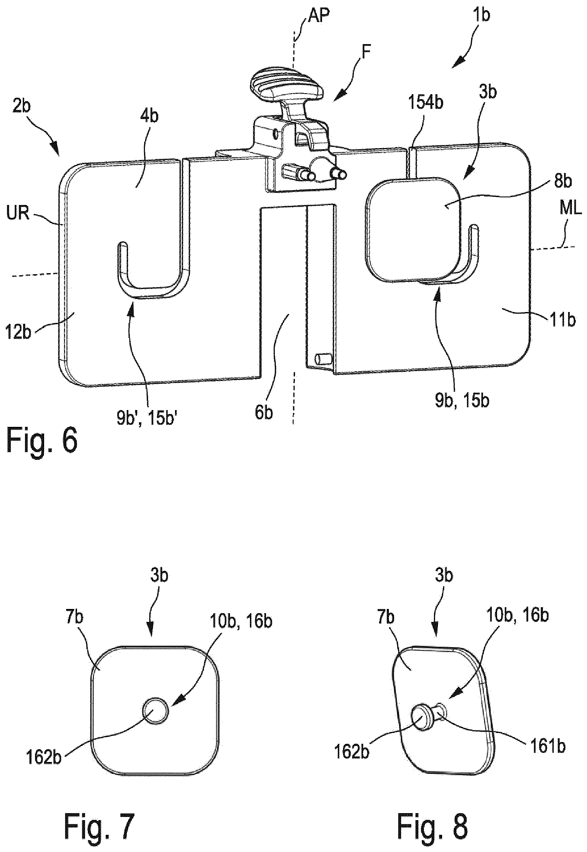

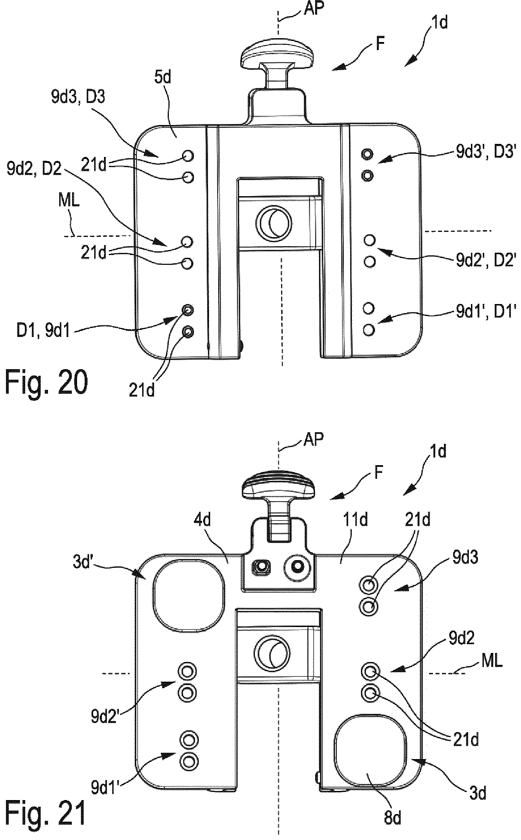

Anhand der Fig. 6 bis 21 sind weitere chirurgische Instrumentensysteme 1b, 1c, 1d gezeigt. Die chirurgischen Instrumentensysteme 1b, 1c, 1d sind hinsichtlich ihrer Funktionsweise und Gestaltung im Wesentlichen übereinstimmend mit dem chirurgischen Instrumentensystem 1a nach den Fig. 1 bis 5. Zur Vermeidung von Wiederholungen wird daher nachfolgend lediglich auf wesentliche Unterschiede der chirurgischen Instrumentensysteme 1b, 1c, 1d gegenüber dem chirurgischen Instrumentensystem 1a nach den Fig. 1 bis 5 eingegangen. Dabei sind funktionsgleiche Bauteile und/oder Abschnitte jeweils mit übereinstimmenden Bezugszeichenziffern unter Hinzufügung eines entsprechenden Kleinbuchstabens gekennzeichnet.

Das chirurgische Instrumentensystem 1b unterscheidet sich im Wesentlichen dadurch, dass der Verbindungsabschnitt 9b ein in die Blockvorderseite 4a eingebrachter und entlang der Anterior-Posterior-Achse AP längserstreckter Kulissenschlitz 15b ist. In diesen Kulissenschlitz 15b greift der komplementäre Verbindungsabschnitt 10b zur unterschiedlichen Positionierung des Kompensationselements 3b in Längsrichtung verschieblich und senkrecht zu der Längsrichtung des Kulissenschlitzes 15b formschlüssig ein.

Bei der gezeigten Ausführungsform ist der Kulissenschlitz 15b abschnittsweise entlang, genauer: parallel zu, der Anterior-Posterior-Achse AP erstreckt. Weiter ist der Kulissenschlitz 15b abschnittsweise entlang der Medial-Lateral-Achse ML erstreckt, wobei vorliegend eine parallele Orientierung vorgesehen ist. Dabei weist der Kulissenschlitz 15b vorliegend im Speziellen eine im weitesten Sinne hakenförmige Längserstreckung auf. Diese ist durch einen ersten Längsabschnitt 151b, einen Querschnitt 152b und einen zweiten Längsabschnitt 153b des Kulissenschlitzes 15b gebildet.

Zudem ist der Kulissenschlitz 15b in Bezug auf seine Längsrichtung einends offen und weist insoweit eine Einführöffnung 154b auf. Die Einführöffnung 154b ist vorliegend auf einer nicht näher bezeichneten Oberseite einer Umfangskontur UR des Referenzblocks 2b angeordnet.

Die Einführöffnung 154b ist zum Einführen des komplementären Verbindungsabschnitts 10b vorgesehen.

Der komplementäre Verbindungsabschnitt 10b ist bei der gezeigten Ausführungsform ein Kulissenstift 16b. Der Kulissenstift 16b ist auf der Elementrückseite 7b angeordnet und ragt entlang deren Normalenrichtung auf. Der Kulissenstift 16b weist einen Schaftabschnitt 161b und einen Kopfabschnitt 162b auf. Der Schaftabschnitt 161b ist hinsichtlich seines nicht näher bezeichneten Durchmessers auf eine nicht näher bezeichnete Schlitzbreite des Kulissenschlitzes 15b abgestimmt. In angebrachtem Zustand des Kompensationselements 3b ist der Schaftabschnitt 161b senkrecht zur Längsrichtung des Kulissenschlitzes 15b und in der Ebene der Blockvorderseite 4b formschlüssig in dem Kulissenschlitz 15b gehalten. In Längsrichtung des Kulissenschlitzes 15b ist der Schaftabschnitt 161b gleitbeweglich.

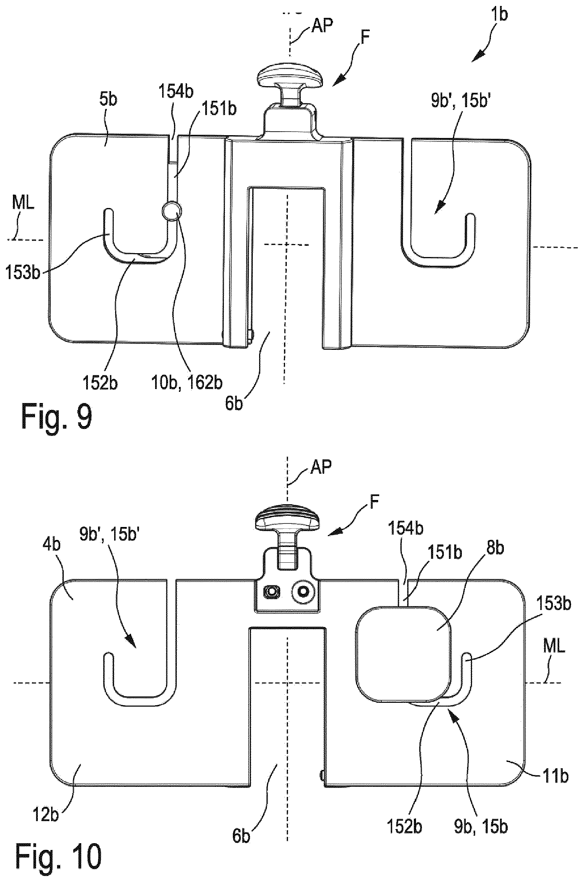

Der Kopfabschnitt 162b ist an einem der Elementrückseite 7b abgewandten Ende des Schaftabschnitts 161b angeordnet. Der Kopfabschnitt 162b weist einen im Vergleich zu dem Schaftabschnitt 161b größeren Durchmesser auf. Der Kopfabschnitt 162b dient einer verliersicheren Anbringung des Kompensationselements in Normalenrichtung der Blockvorderseite 4b. Mit anderen Worten ausgedrückt, hintergreift der Kopfabschnitt 162b die Blockrückseite 5b, so dass das Kompensationselement 3b nicht in Normalenrichtung der Blockvorderseite 4b abgezogen werden kann (Fig. 9).

Zur Anbringung des Kompensationselements 3b an dem Referenzblock 2b wird der Kulissenstift 16b in radialer Richtung des Schaftabschnitts 161b in die Einführöffnung 154b eingeführt. Hierbei hintergreift der Kopfabschnitt 162b den Kulissenschlitz 15b in der zuvor beschriebenen Weise. Zur Positionierung des Kompensationselements 3b wird der Schaftabschnitt 161b entlang der Längsrichtung des Kulissenschlitzes 15b und damit innerhalb des ersten Längsabschnitts 151b und gegebenenfalls entlang des Querabschnitts 152b und/oder des zweiten Längsabschnitts 153b verschoben.

Der Schaftabschnitt 161b weist vorliegend einen kreiszylindrischen Querschnitt auf und ist dementsprechend um seine nicht näher bezeichnete Längsachse relativ zu dem Referenzblock 2b drehbeweglich in dessen Kulissenschlitz 15b gehalten. Hierdurch kann die Kontaktfläche 8b erforderlichenfalls auch rotatorisch angepasst ausgerichtet werden.

Der Kulissenschlitz 15b ist bei der gezeigten Ausführungsform in den medialen Abschnitt 11b der Blockvorderseite 4b eingebracht und in Tiefenrichtung zwischen der Blockvorderseite 4b und der Rückseite 5b durchgängig erstreckt.

Das Verbindungssystem des chirurgischen Instrumentensystems 1b weist vorliegend einen weiteren Verbindungsabschnitt 9b' auf, welcher in den lateralen Abschnitt 12b eingebracht ist. Die Anordnung und/oder Gestaltung des weiteren Verbindungsabschnitts 9b' ist spiegelsymmetrisch und im Übrigen identisch zu dem Verbindungsabschnitt 9b. Dementsprechend ist der weitere Verbindungsabschnitt 9b' ein weiterer Kulissenschlitz 15b'. Das Kompensationselement 3b kann wahlweise auf dem medialen Abschnitt 11b oder dem lateralen Abschnitt 12b angebracht werden.

Im Übrigen versteht sich, dass anstelle der anhand der Fig. 6 bis 10 gezeigten im Wesentlichen quadratischen Umfangskontur des Kompensationselements 3b auch eine kreisförmige oder anderweitig gestaltete Umfangskontur vorgesehen sein kann. Insoweit ist die gezeigte quadratische Umfangskontur als rein exemplarisch zu verstehen. Entsprechendes gilt sinngemäß im Hinblick auf die vorliegend gezeigte hakenförmige Längserstreckung der Kulissenschlitze 15b, 15b'.

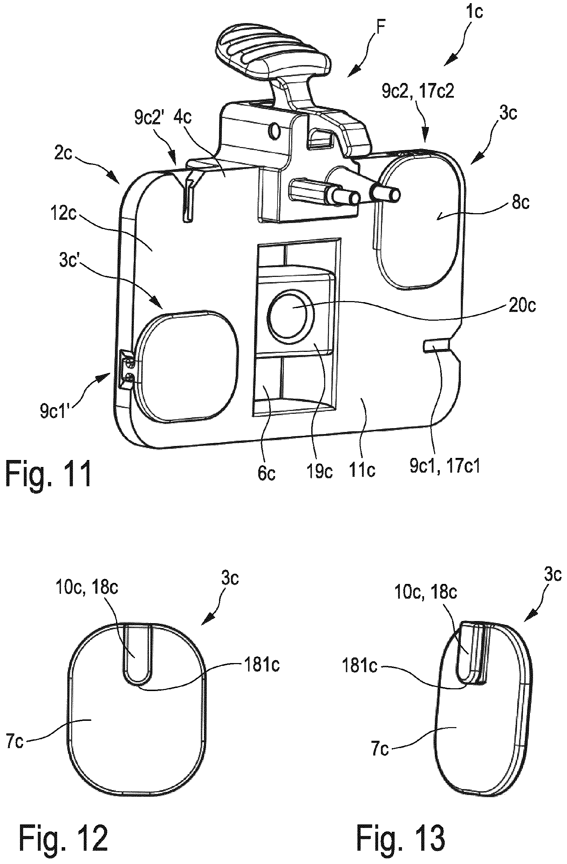

Das chirurgische Instrumentensystem 1c nach den Fig. 11 bis 16 unterscheidet sich im Wesentlichen dadurch von den Instrumentensystemen 1a, 1b, dass mehrere entlang der Anterior-Posterior-Achse AP beabstandet an dem Referenzblock 2c angeordnete Verbindungsabschnitte 9c1, 9c2 vorhanden sind. Zur unterschiedlichen Positionierung des Kompensationselements 3c ist dessen komplementärer Verbindungsabschnitt 10c wahlweise mit einem der beabstandet angeordneten Verbindungsabschnitte 9c1, 9c2 verbindbar.

Bei der gezeigten Ausführungsform weist sowohl der mediale Abschnitt 11c als auch der laterale Abschnitt 12c der Blockvorderseite 4c jeweils mehrere entsprechend in Hochrichtung beabstandete Verbindungsabschnitte auf, die auch als erster medialer Verbindungsabschnitt 9c1, zweiter medialer Verbindungsabschnitt 9c2, erster lateraler Verbindungsabschnitt 9c1' und zweiter lateraler Verbindungsabschnitt 9c2' bezeichnet werden können. Dabei versteht sich, dass mehr als die vorliegend jeweils zwei Verbindungsabschnitte vorhanden sein können, beispielsweise drei, vier, fünf oder gar mehr Verbindungsabschnitte pro Abschnitt der Blockvorderseite 4c.

Die besagten Verbindungsabschnitte definieren jeweils eine Anbringungs-, Schnitt- oder auch Verbindungsstelle zur unterschiedlichen Positionierung des Kompensationselements. Dabei ist das Kompensationselement 3c wie bereits bei den zuvor erläuterten Ausführungsformen wahlweise auf dem medialen Abschnitt 11c oder dem lateralen Abschnitt 12c anbringbar. Die anhand der Fig. 11, 15, 16 gezeigte Konfiguration umfasst neben dem Kompensationselement 3c ein weiteres Kompensationselement 3c', was als rein exemplarisch aufzufassen ist.

Vorliegend sind die mehreren beabstandet angeordneten Verbindungsabschnitte 9c1, 9c2 jeweils als hinterschnittene Nut 17c1 bzw. 17c2 gestaltet. Entsprechendes gilt hinsichtlich der auf dem lateralen Abschnitt 12c angeordneten weiteren Verbindungsabschnitte 9c1', 9c2'.

Die hinterschnittenen Nuten 17c1, 17c2 können auch als erste hinterschnittene Nut 17c1 und zweite hinterschnittene Nut 17c2 bezeichnet werden. Die hinterschnittenen Nuten 17c1, 17c2 unterscheiden sich vorliegend lediglich hinsichtlich ihrer Orientierung, wobei die erste hinterschnittene Nut 17c1' parallel zur Medial-Lateral-Achse ML längserstreckt ist. Die zweite hinterschnittene Nut 17c2 ist parallel zur Anterior-Posterior-Achse AP längserstreckt.

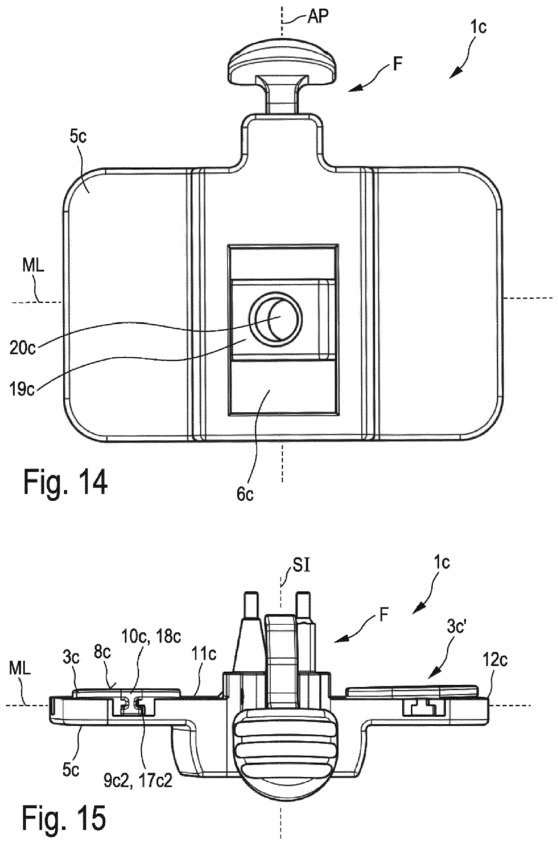

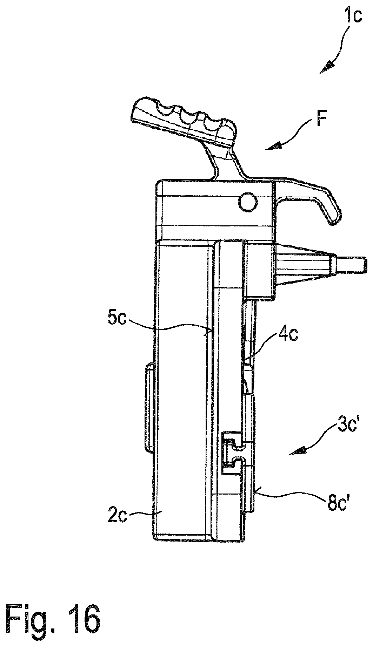

Im Unterschied zu dem Kulissenschlitz 15b der Ausführungsform nach den Fig. 6 bis 10 sind die hinterschnittenen Nuten 17c1, 17c2 nicht durchgängig zwischen der Blockvorderseite 4c und der Blockrückseite 5c erstreckt. Stattdessen sind die hinterschnittenen Nuten 17c1, 17c2 in die Blockvorderseite 4c eingebracht, ohne die Blockrückseite 5c zu durchdringen. Dies ist beispielsweise anhand der Fig. 15, 16 im Detail ersichtlich.

Der komplementäre Verbindungsabschnitt 9c des Kompensationselements 3c ist als Leiste 18c gestaltet. Die Leiste 18c ist auf der Elementrückseite 7c angeordnet und ragt in deren Normalenrichtung auf. Die Leiste 18c weist ein nicht näher bezeichnetes Querschnittsprofil auf, welches mit einem Querschnitt der hinterschnittenen Nuten 17c1, 17c2 korrespondiert. Hierdurch ist das Kompensationselement 3c in Bezug auf die Proximal-Distal-Achse formschlüssig auf der Blockvorderseite anbringbar. Die Leiste 18c weist einen abgerundeten Stirnendabschnitt 181c auf. Der besagte Hinterschnitt der Nuten 17c1, 17c2 ist vorliegend im weitesten Sinne schwalbenschwanzförmig gestaltet. Entsprechendes gilt im Hinblick auf den Querschnitt der Leiste 18c. Die Nuten 17c1, 17c2 und die Leiste 18c bilden eine Art Schwalbenschwanzführung.

Zum Anbringen des Kompensationselements 3c an dem Referenzblock 2c wird die Leiste 18c mit dem abgerundeten Stirnendabschnitt 181c voran in eine der Nuten 17c1, 17c2 auf dem medialen Abschnitt 11c oder eine der entsprechenden Nuten auf dem lateralen Abschnitt 12c eingeschoben. Dies bis eine Endposition erreicht ist, in welcher der abgerundete Stirnendabschnitt 181c an einem nicht näher bezeichneten Ende der jeweiligen Nut anstößt. Zur unterschiedlichen Positionierung des Kompensationselements 3c kann die Leiste 18c wahlweise beispielsweise in die untere erste Nut 17c1 oder die obere zweite Nut 17c2 eingeschoben werden.

Es versteht sich, dass die anhand der Fig. 11 bis 16 ersichtliche Anordnung der hinterschnittenen Nuten 17c1, 17c2 als auch deren Längsorientierung als rein exemplarisch zu verstehen sind. Bei einer nicht gezeigten Ausführungsform sind beispielsweise in Entsprechung zu der ersten hinterschnittenen Nut 17c1 mehrere parallel zu der Medial-Lateral-Achse ML längserstreckte hinterschnittene Nuten über die Höhe des Referenzblocks 2c auf dem medialen Abschnitt 11c verteilt angeordnet. Entsprechendes kann im Hinblick auf den lateralen Abschnitt 12c gelten.

Im Übrigen weist der Referenzblock 2c in Übereinstimmung mit den Referenzblöcken 2a, 2b wiederum einen Durchlass 6c zur Aufnahme eines intramedullären Stabs auf. Der Durchlass 6c ist vorliegend mit einem Einsatzstück 19c versehen. Das Einsatzstück 19c weist eine Aufnahmebohrung 20c zur unmittelbaren Aufnahme des intramedullären Stabs auf. Es versteht sich, dass ein solches Einsatzstück auch bei den Ausführungsformen nach den Fig. 1 bis 10 vorhanden sein kann.

Das Kompensationselement 3c weist eine nicht näher bezeichnete, in etwa rechteckige Umfangskontur mit abgerundeten Ecken auf. Diese Gestaltung ist als rein exemplarisch zu verstehen. Anstelle der abgerundet rechteckigen Umfangskontur kann beispielsweise eine kreisförmige Umfangskontur, wie bei der Ausführungsform nach den Fig. 1 bis 5, oder eine in etwa quadratische Umfangskontur wie bei der Ausführungsform nach den Fig. 6 bis 10 vorgesehen sein.

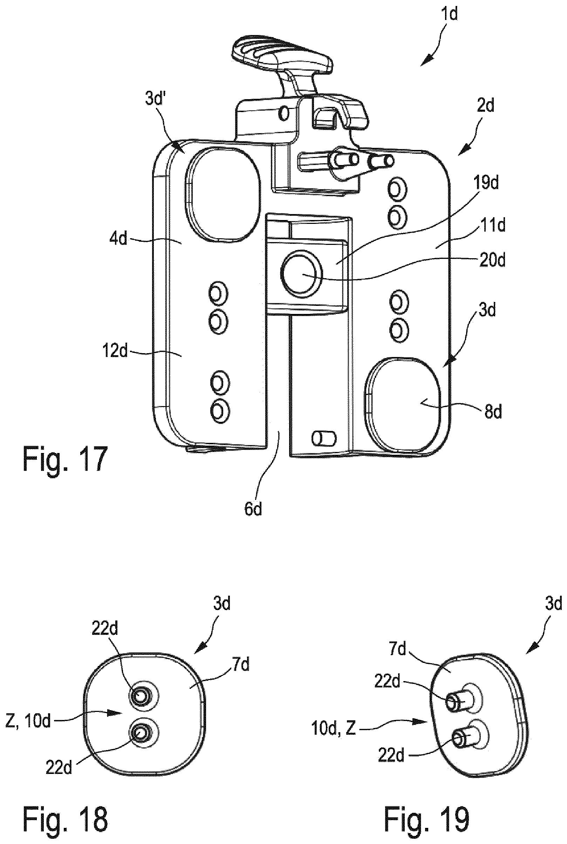

Das anhand der Fig. 17 bis 21 gezeigte chirurgische Instrumentensystem 1d verfügt in Übereinstimmung zu dem chirurgischen Instrumentensystem 1c nach den Fig. 11 bis 16 über mehrere entlang der Anterior-Posterior-Achse AP beabstandet angeordnete Verbindungsabschnitte 9d1, 9d2, 9d3, 9d1', 9d2', 9d3'. Diese sind sowohl auf dem medialen Abschnitt 11d als auch auf dem lateralen Abschnitt 12d der Blockvorderseite 4d angeordnet. Dementsprechend können die mehreren Verbindungsabschnitte auch als erster medialer Verbindungsabschnitt 9d1, zweiter medialer Verbindungsabschnitt 9d2, dritter medialer Verbindungsabschnitt 9d3, erster lateraler Verbindungsabschnitt 9d1', zweiter lateraler Verbindungsabschnitt 9d2' und dritter lateraler Verbindungsabschnitt 9d3' bezeichnet werden.

Bei zeichnerisch nicht dargestellten Ausführungsformen sind auf dem jeweiligen Abschnitt der Blockvorderseite 4d jeweils weniger oder mehr als die vorliegend gezeigten drei Verbindungsabschnitte vorhanden.

Jeder der Verbindungsabschnitte 9d1, 9d2, 9d3, 9d1', 9d2', 9d3' definiert eine Anbringungs- und/oder Verbindungsstelle. In der anhand der Fig. 17, 20, 21 gezeigten Konfiguration ist ein Kompensationselement 3d an dem ersten medialen Verbindungsabschnitt 9d1 angebracht und ein weiteres Kompensationselement 3d' ist an dem dritten lateralen Verbindungsabschnitt 9d3 angebracht. Dies ist wiederum als rein exemplarisch zu verstehen.

Die Verbindungsabschnitte 9d1, 9d2, 9d3, 9d1', 9d2', 9d3' umfassen jeweils wenigstens eine in die Blockvorderseite 4d eingebrachte Bohrung 21d. Die Bohrungen sind vorliegend jeweils als Durchgangsbohrung durchgehend zwischen der Blockvorderseite 4d und der Blockrückseite 5d längserstreckt und parallel zu der Proximal-Distal-Achse SI orientiert.

Der komplementäre Verbindungsabschnitt 10d des Kompensationselements 3d umfasst dementsprechend jeweils wenigstens einen Zapfen 22d, welcher maßlich auf die entsprechenden Bohrungen 21d abgestimmt ist. Der wenigstens eine Zapfen 22d ist auf der Elementrückseite 7d angeordnet und ragt in deren Normalenrichtung auf.

Bei der gezeigten Ausführungsform weist jeder der besagten Verbindungsabschnitte 9d1, 9d2, 9d3, 9d1', 9d2', 9d3' des Referenzblocks 2d mehrere Bohrungen 21d auf. Genauer sind vorliegend exakt zwei Bohrungen 21d je Verbindungsabschnitt vorhanden. Die Bohrungen bilden demnach ein Bohrungspaar. Mit anderen Worten sind die Verbindungsabschnitte 9d1, 9d2, 9d3, 9d1', 9d2', 9d3' jeweils als Bohrungspaar D1, D2, D3, D1', D2' bzw. D3' gestaltet. Die Bohrungen der Bohrungspaare sind bei der gezeigten Ausführungsform in Bezug auf die Anterior-Posterior-Achse AP übereinanderliegend angeordnet. Bei nicht in den Figuren gezeigten Ausführungsformen ist alternativ oder zusätzlich eine nebeneinanderliegende Anordnung vorgesehen.

In Entsprechung zu den Verbindungsabschnitten ist der komplementäre Verbindungsabschnitt 10d als Zapfenpaar Z gestaltet und weist folglich nicht lediglich einen Zapfen, sondern zwei benachbart angeordnete Zapfen 22d auf.

Bei einer zeichnerisch nicht dargestellten Ausführungsform weisen die Verbindungsabschnitte jeweils lediglich eine einzige Bohrung auf und der komplementäre Verbindungsabschnitt weist lediglich einen einzigen Zapfen auf.

Die vorliegende Gestaltung als Bohrungspaar bzw. Zapfenpaar ist besonders robust und wirkt zudem einer ungewollten Rotation des Kompensationselements in Bezug auf die Blockvorderseite entgegen.

## Patentansprüche

1. Chirurgisches Instrumentensystem (1a) zur Verwendung bei einer Kniegelenkersatzoperation, aufweisend
einen Referenzblock (2a), welcher zur Anbringung an einem distalen Femur eingerichtet ist und eine Blockvorderseite (4a) sowie eine entlang einer Proximal-Distal-Achse (SI) gegenüberliegende Blockrückseite (5a) aufweist,
wenigstens ein Kompensationselement (3a) mit einer Elementrückseite (7a) und einer entlang der Proximal-Distal-Achse (SI) gegenüberliegenden Kontaktfläche (8a), welche zur Kontaktierung einer lateralen Kondyle und/oder einer medialen Kondyle des distalen Femurs eingerichtet ist,
und aufweisend ein Verbindungssystem, welches wenigstens einen an dem Referenzblock (2a) angeordneten Verbindungsabschnitt (9a) und wenigstens einen an dem Kompensationselement (3a) angeordneten komplementären Verbindungsabschnitt (10a) aufweist und zur lösbaren Anbringung des Kompensationselements (3a) auf der Blockvorderseite (4a) eingerichtet ist,
wobei das Kompensationselement (3a) die Blockvorderseite (4a) in angebrachtem Zustand wenigstens in Bezug auf eine Anterior-Posterior-Achse (AP) nicht vollständig überdeckt, und dass das Verbindungssystem derart eingerichtet ist, dass das Kompensationselement (3a) mittels des Verbindungssystems wenigstens in Bezug auf die Anterior-Posterior-Achse (AP) in unterschiedlichen Positionen auf der Blockvorderseite (4a) anbringbar ist,
wobei der wenigstens eine Verbindungsabschnitt (9a) eine in die Blockvorderseite (4a) eingebrachte zylindrische Bohrung (13a) ist, **dadurch gekennzeichnet, dass** der wenigstens eine komplementäre Verbindungsabschnitt (10a) ein exzentrisch auf der Elementrückseite (7a) angeordneter zylindrischer Zapfen (14a) ist, welcher in angebrachtem Zustand des Kompensationselements (3a) axial in die Bohrung (13a) eingesteckt ist, wobei das Kompensationselement (3a) aufgrund der exzentrischen Anordnung des Zapfens (14a) mittels einer Rotation um dessen Längsachse (L) wenigstens in Bezug auf die Anterior-Posterior-Achse (AP) unterschiedlich positionierbar ist.

2. Chirurgisches Instrumentensystem (1a) nach Anspruch 1, **dadurch gekennzeichnet, dass** der wenigstens eine Verbindungsabschnitt (9a) an der Blockvorderseite (4a) angeordnet ist, und dass der wenigstens eine komplementäre Verbindungsabschnitt (10a) an der Elementrückseite (7a) angeordnet ist.

3. Chirurgisches Instrumentensystem (1a) nach Anspruch 1 oder 2, **dadurch gekennzeichnet, dass** der wenigstens eine Verbindungsabschnitt (9a) ein in den Referenzblock eingesenkter weiblicher Verbindungsabschnitt ist, und dass der wenigstens eine komplementäre Verbindungsabschnitt (10a) ein von dem Kompensationselement (3a) aufragender männlicher Verbindungsabschnitt ist.

4. Chirurgisches Instrumentensystem (1a) nach einem der vorhergehenden Ansprüche, **dadurch gekennzeichnet, dass** die Blockvorderseite (4a) einen medialen Abschnitt (11a) und einen entlang einer Medial-Lateral-Achse (ML) des Referenzblocks (2a) beabstandeten lateralen Abschnitt (12a) aufweist, welche jeweils wenigstens einen Verbindungsabschnitt (9a) des Verbindungssystems aufweisen, wobei das wenigstens eine Kompensationselement (3a) zur Kontaktierung der lateralen Kondyle auf dem lateralen Abschnitt (12a) und zur Kontaktierung der medialen Kondyle auf dem medialen Abschnitt (11a) anbringbar ist.

5. Chirurgisches Instrumentensystem (1a) nach einem der vorhergehenden Ansprüche, **dadurch gekennzeichnet, dass** das Kompensationselement (3a) und/oder die Kontaktfläche (8a) eine kreisförmige Umfangskontur (U) aufweist.

## Claims

1. Surgical instrument system (1a) for use in a total knee arthroplasty, having
a reference block (2a) which is specified to be attached to a distal femur and has a block front side (4a) and, lying opposite along a proximal-distal axis (SI), a block rear side (5a),
at least one compensation element (3a) which has an element rear side (7a) and, lying opposite along the proximal-distal axis (SI), a contact face (8a) that is specified to contact a lateral condyle and/or a medial condyle of the distal femur,
and having a connection system which has at least one connection portion (9a) that is disposed on the reference block (2a), and at least one complementary connection portion (10a) that is disposed on the compensation element (3a), and which is specified to releasably attach the compensation element (3a) on the block front side (4a),
wherein the compensation element (3a) in the attached state does not completely cover the block front side (4a) at least in terms of an anterior-posterior axis (AP), and in that the connection system is specified in such a manner that the compensation element (3a) by means of the connection system is able to be attached in different positions on the block front side (4a) at least in terms of the anterior-posterior axis (AP),
wherein the at least one connection portion (9a) is a cylindrical bore (13a) incorporated in the block front side (4a),
**characterized in that** the at least one complementary connection portion (10a) is a cylindrical pin (14a) which is disposed eccentrically on the element rear side (7a) and in the attached state of the compensation element (3a) is inserted axially into the bore (13a), wherein the compensation element (3a), owing to the eccentric disposal of the pin (14a), by means of a rotation about the longitudinal axis (L) of the latter is able to be positioned differently at least in terms of the anterior-posterior axis (AP).

2. Surgical instrument system (1a) according to Claim 1, **characterized in that** the at least one connection portion (10a) is disposed on the block front side (4a), and **in that** the at least one complementary connection portion (10a) is disposed on the element rear side (7a).

3. Surgical instrument system (1a) according to Claim 1 or 2, **characterized in that** the at least one connection portion (9a) is a female connection portion recessed in the reference block, and **in that** the at least one complementary connection portion (10a) is a male connection portion protruding from the compensation element (3a).

4. Surgical instrument system (1a) according to one of the preceding claims, **characterized in that** the block front side (4a) has a medial portion (11a) and a lateral portion (12a) that is spaced apart along a medial-lateral axis (ML) of the reference block (2a), which portions have in each case at least one connection portion (9a) of the connection system, wherein the at least one compensation element (3a) for contacting the lateral condyle is able to be attached on the lateral portion (12a), and for contacting the medial condyle is able to be attached on the medial portion (11a).

5. Surgical instrument system (1a) according to one of the preceding claims, **characterized in that** the compensation element (3a) and/or the contact face (8a) have/has a circular circumferential contour (U).

## Revendications

1. Système d'instrument chirurgical (1a) destiné à être utilisé dans une opération de remplacement de l'articulation du genou, présentant
un bloc de référence (2a) adapté pour être installé sur un fémur distal et présentant un côté avant de bloc (4a) ainsi qu'un côté arrière de bloc (5a) opposé le long d'un axe proximal-distal (SI),
au moins un élément de compensation (3a) avec un côté arrière d'élément (7a) et une surface de contact (8a) opposée le long de l'axe proximal-distal (SI), qui est adaptée pour entrer en contact avec un condyle latéral et/ou un condyle médial du fémur distal,
et présentant un système de liaison qui présente au moins une section de liaison (9a) agencée sur le bloc de référence (2a) et au moins une section de liaison complémentaire (10a) agencée sur l'élément de compensation (3a) et qui est adapté pour installer de manière amovible l'élément de compensation (3a) sur le côté avant de bloc (4a),
l'élément de compensation (3a) ne recouvrant pas complètement le côté avant de bloc (4a) à l'état installé, au moins par rapport à un axe antéro-postérieur (AP), et le système de liaison étant adapté de telle sorte que l'élément de compensation (3a) peut être installé au moyen du système de liaison au moins par rapport à l'axe antéro-postérieur (AP) dans différentes positions sur le côté avant de bloc (4a),
l'au moins une section de liaison (9a) étant un alésage cylindrique (13a) pratiqué dans le côté avant de bloc (4a), **caractérisé en ce que** l'au moins une section de liaison complémentaire (10a) est un pivot cylindrique (14a) agencé de manière excentrique sur le côté arrière d'élément (7a), qui, à l'état installé de l'élément de compensation (3a), est inséré axialement dans l'alésage (13a), l'élément de compensation (3a) pouvant être positionné différemment au moins par rapport à l'axe antéro-postérieur (AP) en raison de l'agencement excentrique du pivot (14a) au moyen d'une rotation autour de son axe longitudinal (L).

2. Système d'instrument chirurgical (1a) selon la revendication 1, **caractérisé en ce que** l'au moins une section de liaison (9a) est agencée sur le côté avant de bloc (4a), et **en ce que** l'au moins une section de liaison complémentaire (10a) est agencée sur le côté arrière d'élément (7a).

3. Système d'instrument chirurgical (1a) selon la revendication 1 ou 2, **caractérisé en ce que** l'au moins une section de liaison (9a) est une section de liaison femelle enfoncée dans le bloc de référence, et **en ce que** l'au moins une section de liaison complémentaire (10a) est une section de liaison mâle faisant saillie de l'élément de compensation (3a).

4. Système d'instrument chirurgical (1a) selon l'une quelconque des revendications précédentes, **caractérisé en ce que** le côté avant de bloc (4a) comprend une section médiale (11a) et une section latérale (12a) espacée le long d'un axe médial-latéral (ML) du bloc de référence (2a), qui présentent chacune au moins une section de liaison (9a) du système de liaison, l'au moins un élément de compensation (3a) pouvant être installé pour entrer en contact avec le condyle latéral sur la section latérale (12a) et pour entrer en contact avec le condyle médial sur la section médiale (11a).

5. Système d'instrument chirurgical (1a) selon l'une quelconque des revendications précédentes, **caractérisé en ce que** l'élément de compensation (3a) et/ou la surface de contact (8a) présente un contour périphérique circulaire (U).
